# EUROPEAN PATENT APPLICATION

(11) **EP 3 279 334 A1**
(43) Date of publication of application: **07.02.2018**
(21) Application number: 15887407.3
(22) Date of filing: 30.03.2015
(51) Int. Cl.: C12Q 1/37, C12N 15/00, G01N 33/44, G01N 33/68

(54) **ANIMAL SPECIES DISTINGUISHING METHOD AND DEVICE**

(71) Applicant: National University Corporation Kobe University, Kobe-shi, Hyogo 657-8501 (JP); Kaken Test Center, Tokyo 103-0021 (JP)
(72) Inventor: HATANO, Naoya, Kobe-shi Hyogo 657-8501 (JP); MATOZAKI, Takashi, Kobe-shi Hyogo 657-8501 (JP); IZUCHI, Yukari, Tokyo 103-0021 (JP); TAKASHIMA, Tsuneo, Tokyo 103-0021 (JP)
(74) Representative: Fleck, Hermann-Josef
(86) International application number: PCT/JP2015/001836
(87) International publication number: WO 2016/157250

(57) **Abstract**

To provide an objective method and a device for discriminating animal species by identifying animal species through peptide analysis. This method is provided with: a step for, by using a proteolytic enzyme (protease), cleaving, into peptide fragments, a group of animal fibrous proteins (collagen, etc.) extracted from a discriminating subject sample, and analyzing the peptide fragments through amino acid sequencing; a step for removing, from all the analyzed peptide fragments, peptide fragments commonly detected in all animal species to be discriminated, and selecting peptide fragments that are specifically detected in at least one animal species; and a step for discriminating the animal species of the sample by using the selected peptide fragments. Among the selected peptide fragments, a peptide fragment detected specifically in one particular animal species is used first for discriminating the animal species of the sample, and, with respect to a sample with which discrimination was unsuccessful, a peptide fragment detected specifically in at least two animal species is used next for discriminating the animal species.

## Description

### [Technical Field]

The present invention relates to a technology for objective method of animal species identification, and more particularly to a method for identifying the type of leather.

### [Background Art]

Regarding leather bags, clothing, gloves, and furniture, the quality labelling regulations of miscellaneous manufactured goods are stipulated by the Household Goods Quality Labeling Law. Among those miscellaneous manufactured goods, it is mandatory to discriminate 6 species of animals (a cow, a pig, a sheep, a goat, a horse and a deer) that are display objects for clothes manufactured using leather or synthetic leather for the whole or a part of the products.

In recent years, it is increasingly required that reliable identification of livestock species is performed at the product distribution stage, in order to properly display the quality. However, there are no official laws for such identification at present. Regarding the display concerning these miscellaneous goods, as commonly used identification methods for inspections and tests, an external appearance observation (thickness, size, color and pattern, coat, gloss, feel etc.), a microscope observation (pore arrangement, cross-sectional structure, capillary crust, hair medulla etc.), a device analysis (element, ingredient, dye, drug analysis, etc.) and a DNA identification are listed. (for example, Patent Document 1)

Here, there are problems that the appearance observation requires a long-term experience and is resultantly unconvincing lacking objective and scientific basis. Although the microscope observation is the current mainstream, there are many cases wherein determination becomes unconvincing when the difference among fiber structures or silver surface patterns is inconspicuous, which is a problem. In addition, the instrumental analysis is just an auxiliary means, and identification only by this method is difficult. Regarding a DNA identification, the practical DNA identification of leather has not been implemented although the DNA identification of meat and cereals is widely implemented because of their high precision and high reproducibility. The reason for this is that the DNA is damaged during the untangling process of leather fiber in the leather manufacturing processes such as lime depilating and recalcification immersion, leading to extraction difficulty which sometimes makes the DNA detection impossible. Also, in the case of chromium tanned leather, for example, de-chromium process for oxygen decomposition promotion can rather damage the DNA. As has been described above, the DNA detection is not easy and the method using the DNA identification has not reached to its day to day operation.

As a method based on instrumental analysis, a method for identifying natural leather pieces by pyrolysis gas chromatography / mass spectrometry, which detects thermal decomposition products corresponding to dipeptides derived from collagen is known. (refer to Non-patent literature 1) In this identification method, thermal decomposition products, not easily inhibited by decomposition based on contamination, corresponding to dipeptides derived from Hyp, can be detected as indicator substances identifiable as natural leathers, but their thermal decomposition products do not show any remarkable difference according to animal species, making it difficult to identify the animal species.

### [Prior Art]

### [Patent literature]

[Patent literature 1] JP 2003-125781 A

### [Non-patent literature]

[Non-patent literature 1] Kurata Masaharu, "Pyrolysis gas chromatography / Method for identifying natural leather micro pieces by mass spectrometry", BUNSEKI KAGAKU Vol.57, No.7, pp.563-569 (2008)

### [Outline of the Invention]

### [Problems to be Solved by the Invention]

In view of the above circumstances, it is an object of the present invention to provide an identification method and an identification device capable of conveniently and accurately identifying animal species with respect to an animal leather sample or so on.

### [Means to Solve the Objects]

In order to achieve the above object, the animal species identification method of the present invention comprises a step of analyzing amino acid sequence of peptide fragments after fragmenting the animal fibrous protein group extracted from the identification object sample to peptide fragments by cleavage of the proteolytic enzyme (protease), a step of selecting a peptide fragment specifically detected from at least one animal species after removing the peptide fragments commonly detected for all the animal species to be identified and a step of identifying the animal species of the sample using the selected peptide fragments.

The sample to be identified is leather of 6 animal species such as a cow, a horse, a pig, a sheep, a goat and a deer, and leather of an ostrich, a salmon, a carp, a crocodile and so on, and also fur of a fox, a raccoon, a mink and so on. Fibrous proteins are more difficult to be modified compared with globular proteins and are known to form connective tissues, tendons, bones, skeletal muscles and so on. Examples of fibrous proteins include collagen, keratin, elastin and so on. The amino acid sequence of the peptide fragment after cleaving the fibrous protein group with a proteolytic enzyme to make a peptide fragment and the amino acid sequence of the peptide is consequently analyzed. Trypsin can be suitably used as a proteolytic enzyme, but it is not limited thereto. As a method for analyzing the amino acid sequence of the peptide fragment, a mass spectrometry, a peptide sequencer and a sequence specific peptide antibody and sod on can be suitably used.

In the animal species identification method according to the present invention, the animal species of the sample may be identified using the peptide fragment specifically detected in one specific animal species among the selected peptide fragments, first. And consequently, the animal species may be identified using the peptide fragments specifically detected in at least two species of animal species for samples which had not been identifiable.

First, it becomes possible to identify the animal species of the sample to be identified, efficiently, by identifying the animal species of the sample using peptide fragments specifically detected in one specific animal species. Namely, if there is a peptide fragment specifically detected only in one specific animal species, that one specific kind of animal species can be identified by determining the presence or absence of that peptide fragment and when that peptide fragment is found.

In the step of analyzing the aforementioned amino acid sequence, in the case wherein the analysis is conducted using a mass spectrometry, more specifically, the mass-to-charge ratio (m/z) of precursor ions and product ions are measured by the tandem mass (MS/MS) analysis using a liquid chromatograph -mass spectrometer (LC-MS) and the amino acid sequence is analyzed to identify the peptide fragments.

In the animal species identification method according to the present invention, in the case wherein the method is an identification method of animal skin derived from at least one animal selected from the 6 animal species of a cow, a pig, a sheep, a goat, a horse and a deer, the animal species is identified by determining the presence/absence of at least one peptide fragment among the peptide fragments with the amino acid sequence number 1 to 38, against trypsin decomposed substance of collagen extracted from the sample to be identified.

Namely, 6 species of animal species of a cow, a pig, a sheep, a goat, a horse and a deer are identified by determining the presence or the absence of any peptide fragments among the peptide fragments of GEPGPAGLPGPPGER (SEQ ID NO: 1), GEPGPTGLPGPPGER (SEQ ID NO: 2), GFPGADGVAGPK (SEQ ID NO: 3), GFPGSDGVAGPK (SEQ ID NO: 4), GLTGPIGPPGPAGAPGDKGEAGPSGPAGPTGAR (SEQ ID NO: 5), GLTGPIGPPGPAGAPGDKGETGPSGPAGPTGAR (SEQ ID NO: 6), GETGPAGRPGEVGPPGPPGPAGEK (SEQ ID NO: 7), GETGPAGRPGEAGPPGPPGPAGEK (SEQ ID NO: 8), AGEVGPPGPPGPAGEK (SEQ ID NO: 9), SGDRGETGPAGPAGPIGPVGAR (SEQ ID NO: 10), SGDRGEAGPAGPAGPIGPVGAR (SEQ ID NO: 11), SGDRGETGPAGPAGPVGPVGAR (SEQ ID NO: 12), GIPGPVGAAGATGAR (SEQ ID NO: 13), GIPGPAGAAGATGAR (SEQ ID NO: 14), EGPVGLPGIDGRPGPIGPAGAR (SEQ ID NO: 15), EGPAGLPGIDGRPGPIGPAGAR SEQ ID NO: 16), GEQGPAGPPGFQGLPGPAGTAGEAGKPGER (SEQ ID NO: 17), GEQGPAGPPGFQGLPGPAGTAGEVGKPGER (SEQ ID NO: 18), GIPGEFGLPGPAGAR (SEQ ID NO: 19), GIPGEFGLPGPAGPR (SEQ ID NO: 20), GPPGESGAAGPTGPIGSR (SEQ ID NO: 21), GPPGESGAAGPAGPIGSR (SEQ ID NO: 22), GDGGPPGATGFPGAAGR (SEQ ID NO: 23), GDGGPPGVTGFPGAAGR (SEQ ID NO: 24), GLPGVAGSVGEPGPLGIAGPPGAR (SEQ ID NO: 25), GLPGVAGSLGEPGPLGIAGPPGAR (SEQ ID NO: 26), GEPGPAGAVGPAGAVGPR (SEQ ID NO: 27), GEPGPVGSVGPVGAVGPR (SEQ ID NO: 28), GEPGPAGSVGPAGAVGPR (SEQ ID NO: 29), GEPGPVGAVGPAGAVGPR (SEQ ID NO: 30), IGQPGAVGPAGIR (SEQ ID NO: 31), SGQPGTVGPAGVR (SEQ ID NO: 32), TGQPGAVGPAGIR (SEQ ID NO: 33), GEMGPAGIPGAPGLIGAR (SEQ ID NO: 34), GEMGPAGIPGAPGLMGAR (SEQ ID NO: 35), GAPGPQGPPGAPGPLGIAGLTGAR (SEQ ID NO: 36), GSPGPQGPPGVPGPSGLIGITGAR (SEQ ID NO: 37) and GSPGPQGPPGAPGPGGISGITGAR (SEQ ID NO: 38) which are partial sequences of Collagen Iα1, Collagen I α2, Collagen III α1 (hereinafter abbreviated as col1A1, col1A2, col3A1, respectively).

The peptide fragments of the base SEQ ID NO: 1 to 38 are not the partial sequence numbers common to all 6 animal species regarding COL1A1, COL1A2 and COL3A1, but a partial sequence existing specifically in at least one kind of animal species. The amino acid sequences of the peptide fragments of SEQ ID NO: 1 to 38 have high storage stability among 6 animal species due to the stability of collagen molecules.

The animal species identification method according to the present invention, being an identification method of animal skin of at least one kind of animal-derived skin selected from 6 species of animals of a cow, a pig, a sheep, a goat, a horse and a deer, preferably identifies the animal species by determining the presence or absence of at least any peptide fragments among the peptide fragments of the amino acid sequences of SEQ ID NO: 5 to 14, SEQ ID NO: 19 to 22 and SEQ ID NO: 25 to 33 in trypsin decomposed product of collagen extracted from the sample to be identified.

In the animal species identification method according to the present invention, in the case wherein a cow is an object to be identified, the identification of the presence or the absence of peptide fragments of at least any peptide fragment among peptide fragments of the amino acid sequences of GLTGPIGPPGPAGAPGDKGEAGPSGPAGPTGAR (SEQ ID NO: 5) and IGQPGAVGPAGIR (SEQ ID NO: 31) may be enough to identify the object to be a cow.

The peptide fragments of the SEQ ID NO: 5 and 31 are peptide fragments specifically found only in a cow among the 6 species of animals and a cow can be identified only by these peptide fragments.

In the animal species identification method according to the present invention, in the case wherein a horse is an object to be identified, the identification of the presence or the absence of peptide fragments of at least any peptide fragment among peptide fragments of the amino acid sequences of GEPGPTGLPGPPGER (SEQ ID NO: 2), SGDRGEAGPAGPAGPIGPVGAR (SEQ ID NO: 11), GDGGPPGVTGFPGAAGR (SEQ ID NO: 24), GLPGVAGSLGEPGPLGIAGPPGAR (SEQ ID NO: 26), GEPGPVGSVGPVGAVGPR (SEQ ID NO: 28), SGQPGTVGPAGVR (SEQ ID NO: 32) and GSPGPQGPPGVPGPSGLIGITGAR (SEQ ID NO: 37) may be enough to identify the object to be a horse.

The peptide fragments of the SEQ ID NO: 2, 11, 24, 26, 28, 32 and 37 are peptide fragments specifically found only in a horse among the 6 species of animals of a cow, a pig, a sheep, a goat, a horse and a deer, and a horse can be identified only by these peptide fragments.

In the animal species identification method according to the present invention, in the case wherein a pig is an object to be identified, the identification of the presence or the absence of peptide fragments of at least any peptide fragment among peptide fragments of the amino acid sequences of GETGPAGRPGEAGPPGPPGPAGEK (SEQ ID NO: 8), SGDRGETGPAGPAGPVGPVGAR (SEQ ID NO: 12), GIPGEFGLPGPAGPR (SEQ ID NO: 20), GEPGPAGSVGPAGAVGPR (SEQ ID NO: 29), GEMGPAGIPGAPGLMGAR (SEQ ID NO: 35) and GSPGPQGPPGAPGPGGISGITGAR (SEQ ID NO: 38) may be enough to identify the object to be a pig.

The peptide fragments of the SEQ ID NO: 8, 12, 20, 29, 35 and 38 are peptide fragments specifically found only in a pig among the 6 species of animals of a cow, a pig, a sheep, a goat, a horse and a deer, and a pig can be identified only by these peptide fragments.

In the animal species identification method according to the present invention, in the case wherein a sheep is an object to be identified, the identification of the presence or the absence of peptide fragments of the amino acid sequence of AGEVGPPGPPGPAGEK (SEQ ID NO: 9) may be enough to identify the object to be a sheep.

The peptide fragments of the SEQ ID NO: 9 are peptide fragments specifically found only in a sheep among the 6 species of animals of a cow, a pig, a sheep, a goat, a horse and a deer, and a sheep can be identified only by these peptide fragments.

In the animal species identification method according to the present invention, in a case wherein the animal species of the identification object is narrowed down, the animal species may be identified by narrowing the identification object to be either a cow or a deer after determining the presence or the absence of the amino acid sequences of GEPGPAGAVGPAGAVGPR (SEQ ID NO: 27).

The peptide fragment of the SEQ ID NO: 27 is the peptide fragment specifically found only in a cow and a deer among the 6 species of animals of a cow, a pig, a sheep, a goat, a horse and a deer, and the identification can be narrowed down to either a cow or a deer, or can be identified as a cow or a deer.

In the animal species identification method according to the present invention, in a case wherein the animal species of the identification object is narrowed down, the animal species may be identified by narrowing the identification object to be either a sheep or a goat after determining the presence or the absence of peptide fragments of at least any peptide fragment among peptide fragments of the amino acid sequences of GEPGPVGAVGPAGAVGPR (SEQ ID NO: 30) and GFPGSDGVAGPK (SEQ ID NO: 4).

The peptide fragments of the SEQ ID NO: 4 and 30 are the peptide fragments specifically found only in a sheep and a goat among the 6 species of animals of a cow, a pig, a sheep, a goat, a horse and a deer, and the identification can be narrowed down to either a sheep or a goat, or can be identified as a sheep or a goat.

In the animal species identification method according to the present invention, in a case wherein the animal species of the identification object is narrowed down, the animal species may be identified by narrowing the identification object to be either a horse or a pig after determining the presence or the absence of peptide fragments of at least any peptide fragment among peptide fragments of the amino acid sequences of GIPGPAGAAGATGAR (SEQ ID NO:14) and GEQGPAGPPGFQGLPGPAGTAGEVGKPGER (SEQ ID NO:18).

The peptide fragments of the SEQ ID NO: 14 and 18 are the peptide fragments specifically found only in a horse and a pig among the 6 species of animals of a cow, a pig, a sheep, a goat, a horse and a deer, and the identification can be narrowed down to either a horse or a pig, or can be identified as a horse or a pig.

In the animal species identification method according to the present invention, the identification method being an identification of the animal skin derived from at least one animal selected from the 6 species of animals of a cow, a pig, a sheep, a goat, a horse and a deer, and it is preferable to conduct the steps 1) to 5) below against the trypsin decomposed product of the collagen extracted from the sample to be identified.
1) a step of identifying a cow by determining the presence or absence of peptide fragments of the amino acids with sequences of SEQ ID NO: 5, SEQ ID NO: 27 and SEQ ID NO: 31.
2) a step of identifying a deer by determining the presence or absence of peptide fragments of the amino acids with sequences of SEQ ID NO: 27 and SEQ ID NO: 33.
3) a step of identifying a horse by determining the presence or absence of peptide fragments of the amino acids with sequences of SEQ ID NO: 11 or SEQ ID NO: 28.
4) a step of identifying a pig by determining the presence or absence of peptide fragments of the amino acids with sequences of SEQ ID NO: 12 or SEQ ID NO: 29.
5) a step of identifying a sheep by determining the presence or absence of the peptide fragment of amino acid with the sequence of SEQ ID NO: 9 and a goat by determining the presence or absence of the peptide fragment of amino acid with the sequence of SEQ ID NO: 7 against the remaining samples not yet identified after conducting the steps 1) to 4) above.

Here, the method of determining the presence or absence of peptide fragments can be conducted by determining the presence or absence of peptide fragments by, for example, measuring the mass to charge ratio (m/z) of the precursor ion and the product ion of the peptide fragment using the tandem mass (MS/MS) analysis using a liquid chromatography-mass spectrometer (LC-MS).

In the steps 1) to 5) above, an easy identification of the animal species becomes possible by focusing only on the sum of 5 peaks that are 3 peaks of COL1A1 and 2 peaks of COL1A2 at the measurement result of the mass to charge ratio.

Next, the animal species identification device according to the present invention is described.

The animal species identification device according to the present invention, being a identification device of at least 1 kind of animal skin derived from an animal selected out of 6 species of animals of a cow, a pig, a sheep, a goat, a horse and a deer, is an animal species identification device for identifying the animal species being furnished with means to determine the presence or absence of at least any one of peptide fragments out of peptide fragments having amino acid sequences of SEQ ID No: 1 to 38 against what is obtained by reducing the reduce collagen from the sample to be identified using trypsin.

Moreover, the animal species identification device according to the present invention, being an identification device of animal skin of at least one kind of animal-derived skin selected from 6 species of animals of a cow, a pig, a sheep, a goat, a horse and a deer, is characterized by discriminating animal species by having means for determining the presence or absence of at least any peptide fragments among the peptide fragments of the amino acid sequences of SEQ ID NO: 5 to 14, SEQ ID NO: 19 to 22 and SEQ ID NO: 25 to 33 in trypsin decomposed product of collagen extracted from the sample to be identified.

Here, means for determining the presence or absence of a peptide fragment preferably determines the presence or absence of peptide fragments by measuring a mass-to-charge ratio (m/z) of a precursor ion of a peptide fragment and a product ion using the tandem mass (MS/MS) analysis using a liquid chromatograph-mass spectrometer (LC-MS).

### [Effects of the Invention]

According to the identification method or the identification device of the present invention, it becomes possible to identify the animal species with ease and high accuracy by analyzing the fibrous protein group extracted from animal leather samples and so on, providing effects such as accurate product quality displays of leather products. The identification method according to the present invention can be utilized as an international standard under the circumstance that miscellaneous manufactured goods including clothes are globally traded, not only complementing the microscope method conventionally utilized as the mainstream of animal identification.

### [Brief Description of the Drawings]

[FIG. 1] Figure 1 shows the flow diagram of the animal species identification method according to the present invention.
[FIG. 2] Figure 2 shows the flow diagram (1) of the animal species identification method according to embodiment 1.
[FIG. 3] Figure 3 shows the flow diagram (2) of the animal species identification method according to embodiment 1.

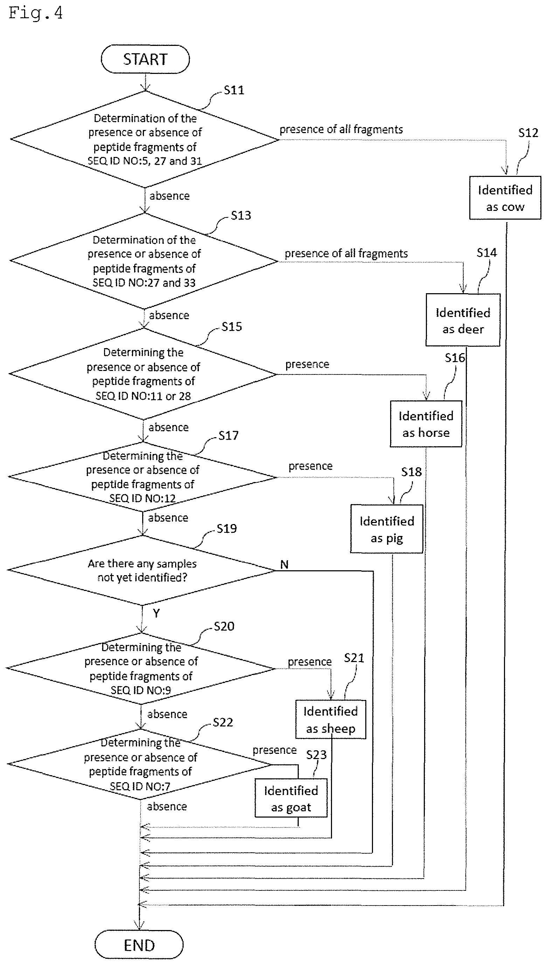

[FIG. 4] Figure 4 shows the flow diagram of 6 animal species identification process.
[FIG. 5] Figure 5 shows the flow diagram of identification process for a cow.
[FIG. 6] Figure 6 shows the flow diagram of identification process for a horse.

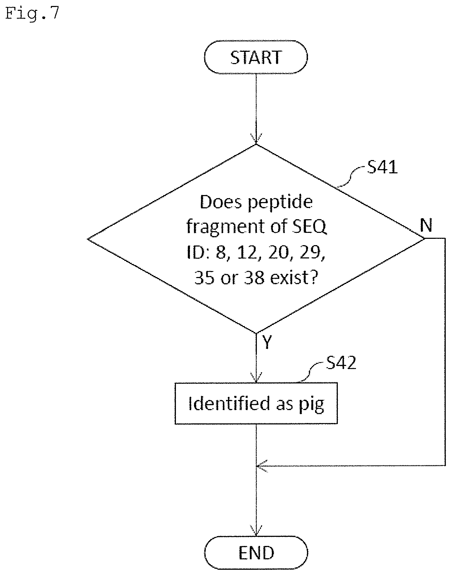

[FIG. 7] Figure 7 shows the flow diagram of identification process for a pig.
[FIG. 8] Figure 8 shows the flow diagram of identification process for a sheep.
[FIG. 9] Figure 9 shows the flow diagram of identification process for a horse or a deer.
[FIG. 10] Figure 10 shows the flow diagram of identification process for a sheep or a goat.
[FIG. 11] Figure 11 shows the flow diagram of identification process for a horse or a pig.

### [Best Mode for Carrying Out the Invention]

Embodiments of the present invention will be described in detail below with reference to the drawings. The present invention is not limited to the following embodiment and examples of shown in the figure, and the present invention can be variously changed in design.

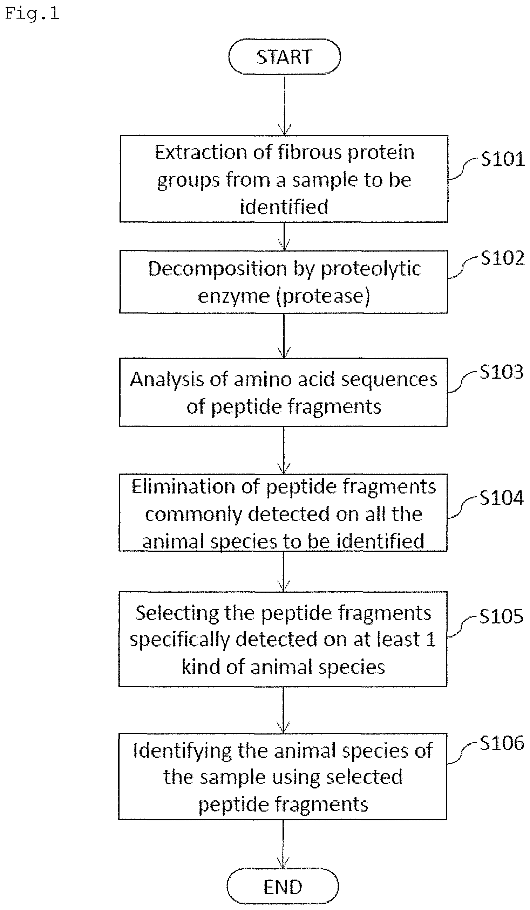

Figure 1 shows the flow diagram of the animal species identification method according to the present invention.

As was described above, the animal species identification method according to the present invention takes the following steps, namely extracting a fibrous protein group from a sample to be identified (step S101), cleaving the fibrous protein groups into peptide fragments by a proteolytic enzyme (protease) (step S102) and analyzing the amino acid sequence of the peptide fragment (step S103). Then, peptide fragments commonly detected in all the animal species to be identified are excluded. (step S104)

Now, a peptide fragment specifically detected in at least 1 animal species is selected (step S105) and the animal species is identified using the peptide fragment so selected. (step S106)

### [Embodiment 1]

According to the embodiment described here, an example of identifying the animal species regarding the natural leather of 6 species of animals of a cow, a pig, a sheep, a goat, a horse and a deer is explained.

First, regarding the animal species of natural leather, collagen which is the tissue and also the main fiber of skin is explained. The animal skin consists of the epidermal layer, the dermal layer and the underlying subcutaneous connective tissue. The epidermal layer consists of 4 layers such as a keratinous layer, a granular layer and so on and these layers are removed because these are useless as leather. Also, elastin fibers in the papillary dermis and the subcutaneous tissues are also mostly removed during mechanical processing and chemical processing in the manufacturing process. Furthermore, the keratin that constitutes hair is unnecessary for the manufacture of leather except for the case of fur pieces and it is decomposed and removed by liming treatment. Therefore, fibers that constitute leather are mostly collagen fibers. The dermis layer is divided into a papillary layer (silver surface layer) and a reticular layer. The upper most layer of the dermis layer corresponds to the grain side layer of leathers and this layer has far finer fibers interwoven minutely than that of the netted layer and many of the fibers run horizontally. Because of this, the silver surface layer of leather is finer compared with the reticular dermis and thus the extension and the moisture permeability worsen.

The amino acid sequence of collagen is more specific compared with those of other proteins. Regarding the amino acid sequence, the characteristics of the primary structure is the repetition of Gly-X-Y and glycine is present at every 3 residues. X and Y are arbitrary amino acids and the frequency of occupying X and Y differs depending on its type. The collagen molecule is formed by twisting the 3 polypeptide chains (α chain) of about 1000 amino acid residues (molecular weight of about 100,000) consisting of such three sets of repetitions. The triple-stranded structure is very strong and not degradable even in enzymes.

There are at least 6 types (I ∼ V types and I type trimmer) of molecular species for constituting the collagen structure. Eight kinds of polypeptide chains are known to constitute the collagen molecule and out of those eight kinds, I type collagen quantitatively amounts the largest, reaching to 80 to 90 % of the total collagen.

Next, the sample for identification used in the experiment is explained. The samples for identification were mainly chosen from chrome-tanned leather of deep and shallow colors, having the largest trade volume, regarding the 6 species of animals (a cow, a pig, a sheep, a goat, a horse and a deer) to be displayed on clothing items following the provisions on industrial household goods of the household goods quality labelling act. It should be noted that the samples dyed with deep color dyes or finished with finishing agents, both being difficult to be extracted by processing and the DNA method, were also used in addition to leathers tanned with tannin to make the leather selection clear without a vague history.

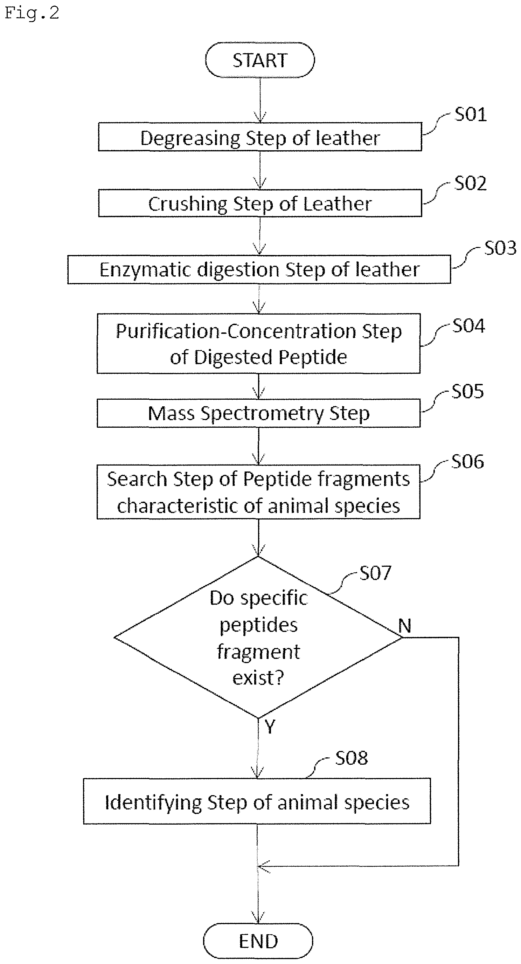

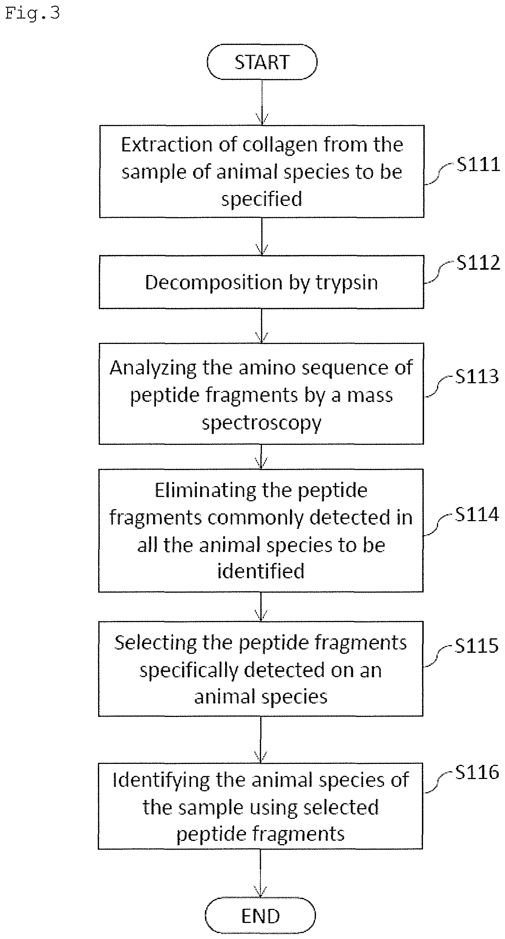

Hereinafter, the flow of the identification method of 6 types of animals regarding the samples to be identified as mentioned above will be explained in detail by referencing the figure 2 and figure 3.

### <Step S01: Degreasing step of leather>

The sample was washed for 2 hours by immersing in diethyl ether (manufactured by Wako Pure Chemical Industries, Ltd.) keeping the circulation more than 6 times per hour. Then the sample was immersed in pure water for 1 hour after being laid so that diethyl ether evaporates. In this step, the bath ratio was set at 100:1 with occasional stirring. After eliminating unnecessary water completely, the sample was laid and dried.

### <Step S02: Crushing step of Leather>

The washed fibers were frozen in liquid nitrogen using a freeze pulverizer (JFC-300, manufactured by Japan Analytical Industry Co., Ltd.) and pulverized for 15 minutes.

### <Step S03: Enzymatic digestion step of leather>

The sample was transferred to tubes, after weighing to 0.5 mg, 500µL of 25mM ammonium hydrogen carbonate (manufactured by Wako Pure Chemical Industries, Ltd.) was added to each tube, treated at 1250 rpm for 1 hour at 60 ° C in an incubator and the tubes were laid for 10 minutes at room temperature. A 20 µg trypsin (Promega, for protein sequencing) was resolved in 25 mM acid ammonium carbonate and the solution was added to each tube. An enzyme reaction was performed at 37 ° C and 250 rpm using an incubator for 18 hours.

### <Step S04: Purification-Concentration step of digested Peptide>

The sample (about 550 µL) was filtrated for 10 minutes at 4900 xg at 4° C using a 0.22µm filter (SUPRECTM-01; manufactured by Takara Bio). After that, the sample was frozen at -80 ° C for 10 minutes and the vacuum-freeze drying at -50 ° C was performed overnight using a freeze dryer (EYELAFDU-1200; manufactured by Tokyo Science Instruments Co., Ltd.).

Next, the sample was dissolved in 0.1% formic acid, vortexed, then slightly centrifuged and sonicated for 5 minutes. The sonicated samples were again filtered using a 0.22 *µ*m filter for 10 minutes at 4900 x g . 2 *µ*L out of the collected samples were used for LC-MS/MS analysis. A formic acid for LCMS was used.

### <Step S 05: Mass spectrometry step>

For mass spectrometry, a high speed liquid chromatographic ion trap type-flight time type mass spectrometer (LCMS-IT-TOF; manufactured by Shimadzu Corporation) and a triple quadrupole LCMS/MS system (G6460 QQQ LC-MS; manufactured by Agilent Technologies) were used.

### <Step S 06: Peptide Fragment search step>

Peptide fragments characteristic of animal species were searched using Error Tolerant Search (a search allowing differences in mass of precursor ions) included in the LCMS-IT-TOF protein identification software (Mascot). In order to make it possible to search for peptides containing hydroxyl-proline and hydroxyl-lysine, cysteine modification (alkylation) was removed from, and not only Oxidation (M) commonly used but also Oxidation (K) were added to, the parameters of protein posttranslational modification.

Results whether some specific peptide fragments for 6 species of animals (a cow, a pig, a sheep, a goat, a horse and a deer) exist or not are described below.

### (A) LCMS-IT-TOF

Three types of collagen (COL1A1, COL1A2 and COL3A1) were mainly detected in the Mascot search conducted after the analysis with LCMS-IT-TOF. Therefore, for each collagen, portions having different amino acid sequences were selected from the 6 animal species. The results of COL1A1, COL1A2 and COL3A1 are shown in Tables 1, 2 and 3, respectively. Note that the notation of the item AA in the table below indicates the amino sequence number corresponding to the amino acid sequence of a cow, the item Ox indicates the total number of amino acids to be oxidatively modified, the item m/z shows the ion mass to charge ratio of the mainly detected ions and the item Mr (calc) shows the theoretical molecular weight (mono isotropic mass).

**[Table 1]**

| SEQ ID NO: | AA | Peptide | Ox | m/z | Mr(calc) | Cow | Horse | Pig | Sheep | Goat | Deer |
|---|---|---|---|---|---|---|---|---|---|---|---|
| 1 | 472-486 | GEPGPAGLPGPPGER | 3 | 718.3 | 1434.6 | ○ | × | ○ | ○ | ○ | ○ |
| 2 | 472-486 | GEPGPTGLPGPPGER | 3 | 733.3 | 1464.6 | × | ○ | × | × | × | × |
| 3 | 493-504 | GFPGADGVAGPK | 1 | 544.7 | 1087.5 | ○ | ○ | O | x | x | O |
| 4 | 493-504 | GFPGSDGVAGPK | 1 | 552.7 | 1103.5 | × | × | × | ○ | ○ | × |
| 5 | 763-795 | GLTGPIGPPGPAGAPGDKGEAGPSGPAGPTGAR | 2 | 951.8 | 2852.4 | ○ | × | × | × | × | × |
| 6 | 763-795 | GLTGPIGPPGPAGAPGDKGETGPSGPAGPTGAR | 2 | 961.8 | 2882.4 | × | ○ | ○ | ○ | ○ | ○ |
| 7 | 910-933 | GETGPAGRPGEVGPPGPPGPAGEK | 3 | 739.3 | 2215.0 | ○ | × | × | × | ○ | ○ |
| 8 | 910-933 | GETGPAGRPGEAGPPGPPGPAGEK | 3 | 730.0 | 2187.0 | × | × | ○ | × | × | × |
| 9 | 918-933 | AGEVGPPGPPGPAGEK | 2 | 724.8 | 1447.6 | × | × | × | ○ | × | × |
| 10 | 1062-108, | SGDRGETGPAGPAGPIGPVGAR | 0 | 659.3 | 1974.9 | ○ | × | × | ○ | ○ | ○ |
| 11 | 1062-1083 | SGDRGEAGPAGPAGPIGPVGAR | 0 | 649.3 | 1944.9 | × | ○ | × | × | × | × |
| 12 | 1062-1083 | SGDRGETGPAGPAGPVGPVGAR | 0 | 654.6 | 1960.9 | × | × | ○ | × | × | × |

**[Table 2]**

| SEQ ID NO: | AA | Peptide | Ox | m/z | Mr(calc) | Cow | Horse | Pig | Sheep | Goat | Deer |
|---|---|---|---|---|---|---|---|---|---|---|---|
| 13 | 326-340 | GIPGPVGAAGATGAR | 1 | 634.3 | 1266.6 | ○ | × | × | ○ | ○ | ○ |
| 14 | 326-340 | GIPGPAGAAGATGAR | 1 | 620.3 | 1238.6 | × | ○ | ○ | × | × | × |
| 15 | 463-484 | EGPVGLPGIDGRPGPIGPAGAR | 1 | 686.0 | 2055.0 | ○ | ○ | × | × | × | O |
| 16 | 463-484 | EGPAGLPGIDGRPGPIGPAGAR | 1 | 676.6 | 2027.0 | × | × | ○ | ○ | ○ | × |
| 17 | 542-571 | GEQGPAGPPGFQGLPGPAGTAGEAGKPGER | 3 | 931.4 | 2791.3 | ○ | × | × | ○ | ○ | × |
| 18 | 542-571 | GEQGPAGPPGFQGLPGPAGTAGEVGKPGER | 3 | 940.7 | 2819.3 | × | ○ | Δ | × | × | × |
| 19 | 572-586 | GIPGEFGLPGPAGAR | 2 | 714.3 | 1426.7 | ○ | ○ | × | ○ | ○ | ○ |
| 20 | 572-586 | GIPGEFGLPGPAGPR | 2 | 727.3 | 1452.7 | × | × | ○ | × | × | × |
| 21 | 590-607 | GPPGESGAAGPTGPIGSR | 1 | 790.8 | 1579.7 | ○ | × | × | ○ | ○ | × |
| 22 | 590-607 | GPPGESGAAGPAGPIGSR | 1 | 775.8 | 1549.7 | × | ○ | ○ | × | × | ○ |
| 23 | 776-792 | GDGGPPGATGFPGAAGR | 2 | 737.3 | 1472.6 | ○ | × | ○ | ○ | ○ | ○ |
| 24 | 776-792 | GDGGPPGVTGFPGAAGR | 2 | 751.3 | 1500.6 | × | ○ | × | × | × | × |
| 25 | 881-904 | GLPGVAGSVGEPGPLGIAGPPGAR | 3 | 711.0 | 2130.1 | ○ | × | ○ | ○ | ○ | ○ |
| | | | 3 | 1066.0 | 2130.1 | | | | | | |
| 26 | 881-904 | GLPGVAGSLGEPGPLGIAGPPGAR | 3 | 715.7 | 2144.1 | × | ○ | × | × | × | × |
| | | | 3 | 1073.0 | 2144.1 | | | | | | |
| 27 | 977-994 | GEPGPAGAVGPAGAVGPR | 1 | 766.8 | 1531.7 | ○ | × | × | × | × | ○ |
| 28 | 977-994 | GEPGPVGSVGPVGAVGPR | 1 | 802.9 | 1603.8 | × | ○ | × | × | × | × |
| 29 | 977-994 | GEPGPAGSVGPAGAVGPR | 1 | 774.8 | 1547.7 | × | × | ○ | × | × | × |
| 30 | 977-994 | GEPGPVGAVGPAGAVGPR | 1 | 780.9 | 1559.8 | × | × | × | O | ○ | × |
| 31 | 1066-1078 | IGQPGAVGPAGIR | 1 | 604.8 | 1207.6 | ○ | × | × | × | × | × |
| 32 | 1066-1078 | SGQPGTVGPAGVR | 0 | 591.8 | 1181.6 | × | ○ | × | × | × | × |
| 33 | 1066-1078 | TGQPGAVGPAGIR | 0 | 590.8 | 1179.6 | × | × | ○ | ○ | ○ | ○ |

**[Table 3]**

| SEQ ID NO: | AA | Peptide | Ox | m/z | Mr(calc) | Cow | Horse | Pig | Sheep | Goat | Deer |
|---|---|---|---|---|---|---|---|---|---|---|---|
| 34 | 396-413 | GEMGPAGIPGAPGLIGAR | 2 | 826.9 | 1651.8 | ○ | ○ | × | ○ | ○ | ○ |
| 35 | 396-413 | GEMGPAGIPGAPGLMGAR | 3 | 851.8 | 1701.7 | × | × | ○ | × | × | × |
| 36 | 933-956 | GAPGPQGPPGAPGPLGIAGLTGAR | 2 | 700.0 | 2097.0 | ○ | × | × | ○ | ○ | × |
| | | | 2 | 1049.5 | 2097.0 | | | | | | |
| 37 | 933-956 | GSPGPQGPPGVPGPSGLIGITGAR | 3 | 725.3 | 2173.1 | × | ○ | × | × | × | × |
| 38 | 933-956 | GSPGPQGPPGAPGPGGISGITGAR | 3 | 1045.5 | 2088.9 | × | × | ○ | × | × | × |

The amino acid sequence of the detected peptide fragment had high preservability among animal species and showed high stability of collagen molecules. However, the partial amino acid sequencing analysis showed slight differences such as one residue (A) and (V).

Note that an amino acid sequence having the same mass-to-charge ratio and the same theoretical molecular weight as SEQ ID NO: 19 exists in the existing database. That is, GLPGEFGLPGPAGAR (SEQ ID NO: 39) of an amino acid sequence different from GIPGEFGLPGPAGAR (SEQ ID NO: 19) exists. This is because leucine (L) and isoleucine (I) have the same molecular weight and there is no difference in mass. Similarly, an amino acid sequence having the same mass to charge ratio and the same theoretical molecular weight as EQ ID NO: 34 exists in the existing database. GEMGPAGIPGAPGLLGAR (SEQ ID NO: 40) of an amino acid sequence different from GEMGPAGIPGAPGLIGAR (SEQ ID NO: 34) exists.

### (B) QQQ LC-MS

Among the peptide fragments identifiable with regard to animal species from the LCMS-IT-TOF analysis results, the peptide fragments stably detected by the QQQ LC-MS analysis are shown in Table 4.

**[Table 4]**

| SEQ 10 NO: | AA | Peptide | Ox | m/z | Mr(calc) | Cow | Horse | Pig | Sheep | Goat | Deer |
|---|---|---|---|---|---|---|---|---|---|---|---|
| 5 | 763-795 | GLTGPIGPPGPAGAPGDKGEAGPSGPAGPTGAR | 2 | 951.8 | 2852.4 | ○ | × | × | × | × | × |
| 6 | 763-795 | GLTGPIGPPGPAGAPGDKGETGPSGPAGPTGAR | 2 | 961.8 | 2882.4 | × | ○ | ○ | ○ | ○ | ○ |
| 7 | 910-933 | GETGPAGRPGEVGPPGPPGPAGEK | 3 | 739.3 | 2215.0 | ○ | × | × | × | ○ | ○ |
| 8 | 910-933 | GETGPAGRPGEAGPPGPPGPAGEK | 3 | 730.0 | 2187.0 | × | × | ○ | × | × | × |
| 9 | 918-933 | AGEVGPPGPPGPAGEK | 2 | 724.8 | 1447.6 | × | × | × | ○ | × | × |
| 10 | 1062-1083 | SGDRGETGPAGPAGPIGPVGAR | 0 | 659.3 | 19 74.9 | ○ | × | × | ○ | ○ | ○ |
| 11 | 1062-1083 | SGDRGEAGPAGPAGPIGPVGAR | 0 | 649.3 | 1944.9 | × | ○ | × | × | × | × |
| 12 | 1062-1083 | SGDRGETGPAGPAGPVGPVGAR | 0 | 654.6 | 1960.9 | × | × | ○ | × | × | × |
| 13 | 326-340 | GIPGPVGAAGATGAR | 1 | 634.3 | 1266.6 | ○ | × | × | ○ | ○ | ○ |
| 14 | 326-340 | GIPGPAGAAGATGAR | 1 | 620.3 | 1238.6 | × | ○ | ○ | × | × | × |
| 19 | 572-586 | GIPGEFGLPGPAGAR | 2 | 714.3 | 1426.7 | ○ | ○ | × | ○ | ○ | ○ |
| 20 | 572-586 | GIPGEFGLPGPAGPR | 2 | 727.3 | 1452.7 | × | × | ○ | × | × | × |
| 21 | 590-607 | GPPGESGAAGPTGPIGSR | 1 | 790.8 | 1579.7 | ○ | × | × | ○ | ○ | × |
| 22 | 590-607 | GPPGESGAAGPAGPIGSR | 1 | 775.8 | 1549.7 | × | ○ | ○ | × | × | ○ |
| 25 | 881-904 | GLPGVAGSVGEPGPLGIAGPPGAR | 3 | 711.0 | 2130.1 | ○ | × | ○ | ○ | ○ | ○ |
| | | | 3 | 1066.0 | 2130.1 | | | | | | |
| 26 | 881-904 | GLPGVAGSLGEPGPLGIAGPPGAR | 3 | 715.7 | 2144.1 | × | ○ | × | × | × | × |
| | | | 3 | 1073.0 | 2144.1 | | | | | | |
| 27 | 977-994 | GEPGPAGAVGPAGAVGPR | 1 | 766.8 | 1531.7 | ○ | × | × | × | × | ○ |
| 28 | 977-994 | GEPGPVGSVGPVGAVGPR | 1 | 802.9 | 1603.8 | × | ○ | × | × | × | × |
| 29 | 977-994 | GEPGPAGSVGPAGAVGPR | 1 | 774.8 | 1547.7 | × | × | ○ | × | × | × |
| 30 | 977-994 | GEPGPVGAVGPAGAVGPR | 1 | 780.9 | 1559.8 | × | × | × | ○ | ○ | × |
| 31 | 1066-1078 | IGQPGAVGPAGIR | 1 | 604.8 | 1207.6 | ○ | × | × | × | × | × |
| 32 | 1066-1078 | SGQPGTVGPAGVR | 0 | 591.8 | 1181.6 | × | ○ | × | × | × | × |
| 33 | 1066-1078 | TGQPGAVGPAGIR | 0 | 590.8 | 1179.6 | × | × | ○ | ○ | ○ | ○ |

When the animal species differentiation test using leather by the QQQ LC-MS is routinely conducted, the identification of the animal species can be simplified by further narrowing down the specific peptide fragments shown in Table 4.

Examples of procedures for identifying the leather of 6 species of animals of a cow, a pig, a sheep, a goat, a horse and a deer are described as below. In case of the leather of 6 species of animals of a cow, a pig, a sheep, a goat, a horse and a deer, it is easily possible to identify the animal species by paying attention only to 5 peaks in total of 3 peaks of COL1A1 and 2 peaks of COL1A2.
(1) First of all, a cow is identified by focusing on m/z951.8, 604. 8, and 767 specifically detected in a cow which has the largest distribution volume.
(2) At this time, m/z767 is detected commonly also in a deer but m/z590.8 is also specific and a deer is identified by the presence or absence of detection of these.
(3) A horse is identified by focusing on specific m/z 649.3 or m/z 802.9. Note that the presence or absence of m/z591.8 may also be confirmed.
(4) A pig is identified by focusing on specific m/z 654.7 or m/z 774.8.
(5) A sheep and a goat are identified focusing on the difference that a sheep shows the specific m/z724.9 and a goat shows the specific m/z739.3.

The results of animal species identification using the identification procedures (1) to (5) above and the identification results using LCMS-IT-TOF, regarding 6 species of animals (a cow, a pig, a sheep, a goat, a horse and a deer), are compared. 3∼5 types of samples for each animal species were prepared to evaluate the accuracy of the analysis and it was learned that the accuracy of this analysis was as high as almost 100 %.

According to the analysis based on the DNA identification method and also the MALDI-TOF MS, the identification is difficult to achieve due to the damage by the chemical agents at the leather processing steps and also due to contaminants such as dyes and tanning agents. In the animal species identification method according to the present invention, these problems mentioned above are solved and there is a high possibility of practical use.

### [Embodiment 2]

In the present embodiment, examples of identifying animal species are explained regarding furs such as a fox, a raccoon, a mink so on. The flow of the animal species identification method for furs mentioned above follows the flow of Figure 1 similarly as in the first embodiment.

First, keratin is extracted from furs such as a fox, a raccoon, a mink so on (Step S101). The extracted keratin is degraded with a keratinase (Step S102). Trypsin can be used as the keratinase.

And then, similarly as in the embodiment 1, the amino acid sequence of the peptide fragment is analyzed (Step S103) to exclude peptide fragments commonly detected (Step S104) in all animal species, and peptide fragments specifically detected in animal species concerned are selected (Step S105). And, the animal species is identified using the selected peptide fragments (Step S106).

### (Other embodiments)

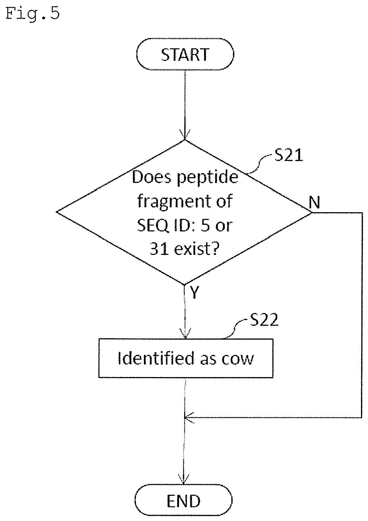

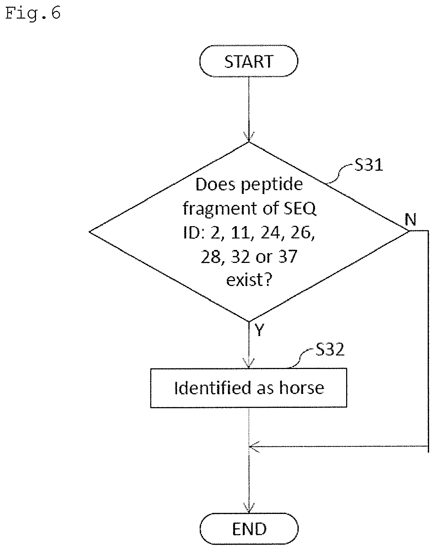

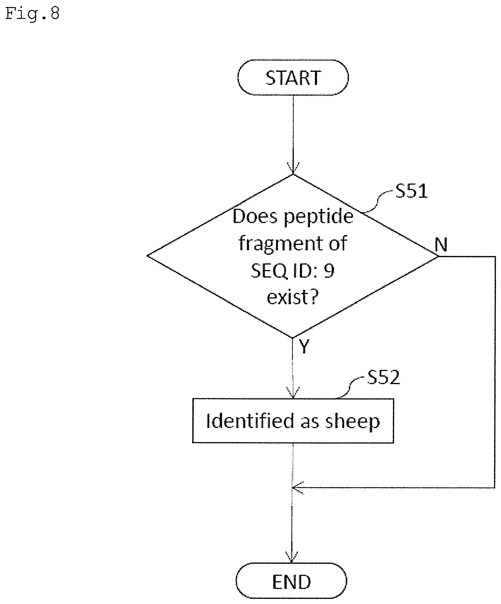

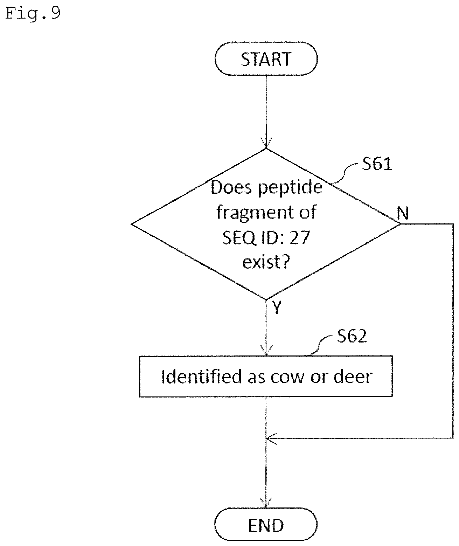

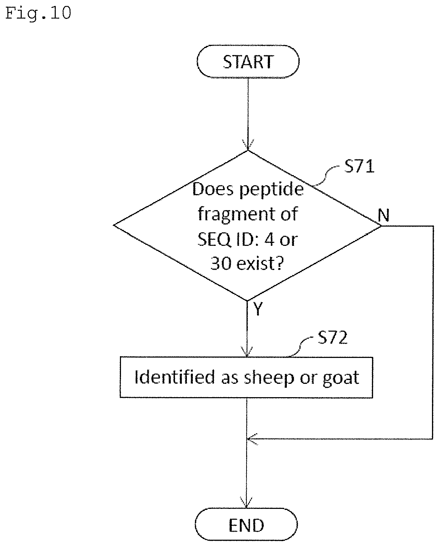

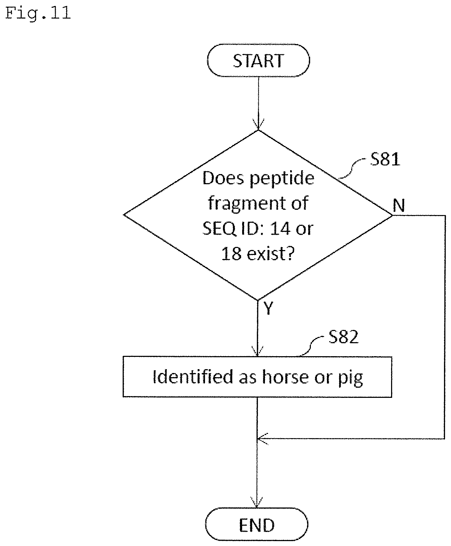

(1) When the identification target is a cow, as shown in Figure 5, the presence or absence of the peptide fragments of the amino acid sequences of SEQ ID NO: 5 or SEQ ID NO: 31 is discriminated to identify a cow.
(2) When the identification target is a horse, as shown in figure 6, the presence or absence of the peptide fragments of the amino acid sequences of SEQ ID NO: 2, SEQ ID NO: 11, SEQ ID NO: 24, SEQ ID NO: 26, SEQ ID NO: 28, SEQ ID NO: 32 or SEQ ID NO: 37 is discriminated to identify a horse.
(3) When the identification target is a pig, the presence or absence of peptide fragments of the amino acid sequences of SEQ ID NO: 8, SEQ ID NO: 12, SEQ ID NO: 20, SEQ ID NO: 29, SEQ ID NO: 35 or SEQ ID NO: 38 is distinguished to identify a pig.
(4) When the identification target is a sheep, as shown in Figure 8, the presence or absence of a peptide fragment having the amino acid sequence of SEQ ID NO: 9 is discriminated to identify a sheep.
(5) When narrowing down the animal species to be identified, as shown in Figure 9, the presence or absence of the peptide fragment of the amino acid sequence of SEQ ID NO: 27 can be discriminated and the object to be identified can be narrowed down to a cow or a deer.
(6) When narrowing down the animal species to be identified, as shown in Figure 10, the presence or absence of the peptide fragment of the amino acid sequence of SEQ ID NO: 4 or SEQ ID NO: 30 can be discriminated and the object to be identified can be narrowed down to a sheep or a goat.
(7) When narrowing down the animal species to be identified, as shown in Figure 11, the presence or absence of a peptide fragment of the amino acid sequence of SEQ ID NO: 14 or SEQ ID NO: 18 can be discriminated and the object to be identified can be narrowed down to a horse or a pig.

### [Industrial Applicability]

The present invention is useful for identifying an animal species of natural leather.

\201@\201@\201@\201@\201@\201@\201@\201@\201@ \201@\201@\201@\201@SEQUENCE
LISTING

<110> NATIONAL UNIVERSITY CORPORATION KOBE UNIVERSITY; KAKEN TEST CENTER
<120> Animal species differentiation method and apparatus
<160> 40
<210> 1
<211> 15
<212> PRT
<213> Bos taurus
<400> 1
Gly Glu Pro Gly Pro Ala Gly Leu Pro Gly Pro Pro Gly Glu Arg
1               5                   10                  15

<210> 2
<211> 15
<212> PRT
<213> Equus caballus
<400> 2
Gly Glu Pro Gly Pro Thr Gly Leu Pro Gly Pro Pro Gly Glu Arg
1               5                   10                  15

<210> 3
<211> 12
<212> PRT
<213> Bos taurus
<400> 3
Gly Phe Pro Gly Ala Asp Gly Val Ala Gly Pro Lys
1               5                   10

<210> 4
<211> 12
<212> PRT
<213> Capra Linnaeus
<400> 4
Gly Phe Pro Gly Ser Asp Gly Val Ala Gly Pro Lys
1               5                   10

<210> 5
<211> 33
<212> PRT
<213> Bos taurus
<400> 5
Gly Leu Thr Gly Pro Ile Gly Pro Pro Gly Pro Ala Gly Ala Pro Gly
1               5                   10
15\201@\201@\201@\201@\201@\201@
Asp Lys Gly Glu Ala Gly Pro Ser Gly Pro Ala Gly Pro Thr Gly Ala
\201@\201@\201@      20\201@\201@\201@\201@\201@\201@\201@\201@\201@25
30
Arg


<210> 6
<211> 33
<212> PRT
<213> Ovis aries
<400> 6
Gly Leu Thr Gly Pro Ile Gly Pro Pro Gly Pro Ala Gly Ala Pro Gly
1               5                   10
15\201@\201@\201@\201@\201@\201@
Asp Lys Gly Glu Thr Gly Pro Ser Gly Pro Ala Gly Pro Thr Gly Ala
\201@      \201@\201@20\201@\201@\201@\201@\201@\201@\201@\201@\201@25
30

Arg

<210> 7
<211> 24
<212> PRT
<213> Bos taurus
<400> 7
Gly Glu Thr Gly Pro Ala Gly Arg Pro Gly Glu Val Gly Pro Pro Gly
1               5                   10                  15\201@\201@\201@
Pro Pro Gly Pro Ala Gly Glu Lys
            20

<210> 8
<211> 24
<212> PRT
<213> Sus scrofa domesticus
<400> 8
Gly Glu Thr Gly Pro Ala Gly Arg Pro Gly Glu Ala Gly Pro Pro Gly
1               5                   10                  15\201@\201@\201@
Pro Pro Gly Pro Ala Gly Glu Lys
\201@\201@\201@\201@\201@\201@20

<210> 9
<211> 16
<212> PRT
<213> Ovis aries
<400> 9
Ala Gly Glu Val Gly Pro Pro Gly Pro Pro Gly Pro Ala Gly Glu Lys
1               5                   10                  15

<210> 10
<211> 22
<212> PRT
<213> Bos taurus
<400> 10
Ser Gly Asp Arg Gly Glu Thr Gly Pro Ala Gly Pro Ala Gly Pro Ile
1               5                   10                  15\201@\201@\201@
Gly Pro Val Gly Ala Arg
\201@\201@\201@\201@\201@\201@20

<210> 11
<211> 22
<212> PRT
<213> Equus caballus
<400> 11
Ser Gly Asp Arg Gly Glu Ala Gly Pro Ala Gly Pro Ala Gly Pro Ile
1               5                   10                  15\201@\201@\201@
Gly Pro Val Gly Ala Arg\201@\201@\201@\201@\201@\201@      20

<210> 12
<211> 22
<212> PRT
<213> Sus scrofa domesticus
<400> 12
Ser Gly Asp Arg Gly Glu Thr Gly Pro Ala Gly Pro Ala Gly Pro Val
1               5                   10                  15\201@\201@\201@
Gly Pro Val Gly Ala Arg
\201@\201@\201@\201@\201@\201@20

<210> 13
<211> 15
<212> PRT
<213> Bos taurus

<400> 13
Gly Ile Pro Gly Pro Val Gly Ala Ala Gly Ala Thr Gly Ala Arg
1               5                   10                  15

<210> 14
<211> 15
<212> PRT
<213> Equus caballus
<400> 14
Gly Ile Pro Gly Pro Ala Gly Ala Ala Gly Ala Thr Gly Ala Arg
1               5                   10                  15

<210> 15
<211> 22
<212> PRT
<213> Bos taurus
<400> 15
Glu Gly Pro Val Gly Leu Pro Gly Ile Asp Gly Arg Pro Gly Pro Ile
1               5                   10                  15\201@\201@\201@
Gly Pro Ala Gly Ala Arg
\201@\201@\201@\201@\201@\201@20

<210> 16
<211> 22
<212> PRT
<213> Sus scrofa domesticus
<400> 16
Glu Gly Pro Ala Gly Leu Pro Gly Ile Asp Gly Arg Pro Gly Pro Ile
1               5                   10                  15\201@\201@\201@
Gly Pro Ala Gly Ala Arg
      \201@\201@\201@20

<210> 17
<211> 30
<212> PRT
<213> Bos taurus
<400> 17
Gly Glu Gln Gly Pro Ala Gly Pro Pro Gly Phe Gln Gly Leu Pro Gly
1               5                   10                  15\201@\201@\201@
Pro Ala Gly Thr Ala Gly Glu Ala Gly Lys Pro Gly Glu Arg
\201@\201@\201@\201@\201@\201@20\201@\201@\201@\201@\201@\201@\201@\201@\201@
25                  30

<210> 18
<211> 30
<212> PRT
<213> Equus caballus
<400> 18
Gly Glu Gln Gly Pro Ala Gly Pro Pro Gly Phe Gln Gly Leu Pro Gly
1               5                   10                  15\201@\201@\201@
Pro Ala Gly Thr Ala Gly Glu Val Gly Lys Pro Gly Glu Arg
\201@\201@\201@\201@\201@\201@20\201@\201@\201@\201@\201@\201@\201@\201@\201@
25                  30

<210> 19
<211> 15
<212> PRT
<213> Bos taurus
<400> 19
Gly Ile Pro Gly Glu Phe Gly Leu Pro Gly Pro Ala Gly Ala Arg
1               5                   10                  15

<210> 20

<211> 15
<212> PRT
<213> Sus scrofa domesticus
<400> 20
Gly Ile Pro Gly Glu Phe Gly Leu Pro Gly Pro Ala Gly Pro Arg
1               5                   10                  15

<210> 21
<211> 18
<212> PRT
<213> Bos taurus
<400> 21
Gly Pro Pro Gly Glu Ser Gly Ala Ala Gly Pro Thr Gly Pro Ile Gly
1               5                   10                  15\201@\201@\201@
Ser Arg


<210> 22
<211> 17
<212> PRT
<213> Equus caballus
<400> 22
Gly Pro Gly Glu Ser Gly Ala Ala Gly Pro Ala Gly Pro Ile Gly Ser
1               5                   10                  15\201@\201@\201@
Arg


<210> 23
<211> 17
<212> PRT
<213> Bos taurus
<400> 23
Gly Asp Gly Gly Pro Pro Gly Ala Thr Gly Phe Pro Gly Ala Ala Gly
1               5                   10                  15\201@\201@\201@
Arg


<210> 24
<211> 17
<212> PRT
<213> Equus caballus
<400> 24
Gly Asp Gly Gly Pro Pro Gly Val Thr Gly Phe Pro Gly Ala Ala Gly
1               5                   10                  15\201@\201@\201@
Arg


<210> 25
<211> 24
<212> PRT
<213> Bos taurus
<400> 25
Gly Leu Pro Gly Val Ala Gly Ser Val Gly Glu Pro Gly Pro Leu Gly
1               5                   10                  15\201@\201@\201@
Ile Ala Gly Pro Pro Gly Ala Arg
\201@\201@\201@\201@\201@\201@20

<210> 26
<211> 24
<212> PRT
<213> Equus caballus
<400> 26
Gly Leu Pro Gly Val Ala Gly Ser Leu Gly Glu Pro Gly Pro Leu Gly

1                   5                   10                  15\201@\201@\201@
Ile Ala Gly Pro Pro Gly Ala Arg
\201@\201@  \201@\201@\201@20

<210> 27
<211> 18
<212> PRT
<213> Cervidae Goldfuss
<400> 27
Gly Glu Pro Gly Pro Ala Gly Ala Val Gly Pro Ala Gly Ala Val Gly
1                   5                   10                  15\201@\201@\201@
Pro Arg

<210> 28
<211> 18
<212> PRT
<213> Equus caballus
<400> 28
Gly Glu Pro Gly Pro Val Gly Ser Val Gly Pro Val Gly Ala Val Gly
1                   5                   10                  15\201@\201@\201@
Pro Arg

<210> 29
<211> 18
<212> PRT
<213> Sus scrofa domesticus
<400> 29
Gly Glu Pro Gly Pro Ala Gly Ser Val Gly Pro Ala Gly Ala Val Gly
1                   5                   10                  15\201@\201@\201@
Pro Arg

<210> 30
<211> 18
<212> PRT
<213> Capra Linnaeus
<400> 30
Gly Glu Pro Gly Pro Val Gly Ala Val Gly Pro Ala Gly Ala Val Gly
1                   5                   10                  15\201@\201@\201@
Pro Arg

<210> 31
<211> 13
<212> PRT
<213> Bos taurus
<400> 31
Ile Gly Gln Pro Gly Ala Val Gly Pro Ala Gly Ile Arg
1                   5                   10

<210> 32
<211> 13
<212> PRT
<213> Equus caballus
<400> 32
Ser Gly Gln Pro Gly Thr Val Gly Pro Ala Gly Val Arg
1                   5                   10

<210> 33
<211> 13
<212> PRT

<213> Sus scrofa domesticus
<400> 33
Thr Gly Gln Pro Gly Ala Val Gly Pro Ala Gly Ile Arg
1               5                   10

<210> 34
<211> 18
<212> PRT
<213> Bos taurus
<400> 34
Gly Glu Met Gly Pro Ala Gly Ile Pro Gly Ala Pro Gly Leu Ile Gly
1               5                   10                  15\201@\201@\201@
Ala Arg


<210> 35
<211> 18
<212> PRT
<213> Sus scrofa domesticus
<400> 35
Gly Glu Met Gly Pro Ala Gly Ile Pro Gly Ala Pro Gly Leu Met Gly
1               5                   10                  15\201@\201@\201@
Ala Arg


<210> 36
<211> 24
<212> PRT
<213> Bos taurus
<400> 36
Gly Ala Pro Gly Pro Gln Gly Pro Pro Gly Ala Pro Gly Pro Leu Gly
1               5                   10                  15\201@\201@\201@
ILE Ala Gly Leu Thr Gly Ala Arg
\201@\201@\201@\201@\201@\201@20

<210> 37
<211> 24
<212> PRT
<213> Equus caballus
<400> 37
Gly Ser Pro Gly Pro Gln Gly Pro Pro Gly Val Pro Gly Pro Ser Gly
1               5                   10                  15\201@\201@\201@
Leu Ile Gly Ile Thr Gly Ala Arg
\201@\201@\201@\201@\201@\201@20

<210> 38
<211> 24
<212> PRT
<213> Sus scrofa domesticus
<400> 38
Gly Ser Pro Gly Pro Gln Gly Pro Pro Gly Ala Pro Gly Pro Gly Gly
1               5                   10                  15\201@\201@\201@
Ile Ser Gly Ile Thr Gly Ala Arg
\201@\201@\201@\201@\201@\201@20

<210> 39
<211> 15
<212> PRT
<213> Bos taurus
<400> 39
Gly Leu Pro Gly Glu Phe Gly Leu Pro Gly Pro Ala Gly Ala Arg
1               5                   10                  15

<210> 40
<211> 18
<212> PRT
<213> Bos taurus
<400> 40
Gly Glu Met Gly Pro Ala Gly Ile Pro Gly Ala Pro Gly Leu Leu Gly
1               5                   10                  15\201@\201@\201@
Ala Arg

## Claims

1. An animal species identification method, comprising:
a step of analyzing an amino acid sequence of a peptide fragment after cutting the animal fibrous protein group extracted from a sample to be identified with a proteolytic enzyme (protease) into a peptide fragment;
a step of preparing in advance the sequence data group selected peptide fragments specifically detected in at least one species of animal species after removing the peptide fragments commonly detected in all animal species to be identified from all the analyzed peptide fragments; and
a step of discriminating the animal species of the sample using the peptide fragments contained in the selected sequence data group.

2. An animal species identification method as set forth in claim 1, wherein the animal species of the sample is discriminated from the selected peptide fragments based on the peptide fragments specifically detected only in one specific animal species.

3. An animal species identification method as set forth in claim 1, wherein the animal species of the sample is discriminated by identifying the animal species being composed of the step of identifying an animal species of the sample based on the peptide fragment specifically detected only in one specific animal species and further identifying the animal species, for the sample not yet identified, employing the peptide fragment specifically detected in at least two or more kinds of animal species.

4. An animal species identification method as set forth in either one of claims 1 to 3, further comprising:
said animal fibrous protein group being collagen.

5. An animal species identification method as set forth in either one of claims 1 to 4, further comprising:
said proteolytic enzyme being trypsin.

6. An animal species identification method as set forth in either one of claims 1 to 5, further comprising:
said step of analyzing the amino acid sequence of peptide fragments being analyzed using mass spectrometry.

7. An animal species identification method as set forth in either one of claims 1 to 6, further comprising:
said mass analysis being an identification of a peptide fragment by an analysis of an amino acid sequence by measuring the mass-to-charge ratio (m/z) of the pre-cursor ion and the product ion of the peptide fragment by the tandem mass (MS/MS) analysis employing a liquid chromatography mass spectrometer (LC-MS).

8. An animal species identification method as set forth in either one of claims 1 to 7, further comprising:
said sample to be identified being an animal skin derived from at least one kind of animal selected from six kinds of animals that are a cow, a horse, a pig, a sheep, a goat and a deer.

9. An animal species identification method being an identification of animal skin derived from at least one kind of animal selected from six kinds of animals of a cow, a pig, a sheep, a goat, a horse and a deer, comprising:
identifying the animal species by determining the presence or absence of at least any peptide fragments among peptide fragments of amino acids with sequences of SEQ ID NO: 1∼38 against the decomposition product of animal skin-derived collagen extracted from a sample to be decomposed using trypsin.

10. An animal species identification method as set forth in claim 9, further comprising:
identification of an animal species by determining the presence or absence of at least either one peptide fragment among the peptide fragments of amino acids with sequences of SEQ ID NO: 5∼14, SEQ ID NO: 19∼22 and SEQ ID NO: 25∼33.

11. An animal species identification method as set forth in claim 8 or 9, further comprising:
identification of an animal species by determining the presence or absence of at least either one peptide fragment among the peptide fragments of amino acids with sequences of GLTGPIGPPGPAGAPGDKGEAGPSGPAGPTGAR (SEQ ID NO:5) and IGQPGAVGPAGIR (SEQ ID NO:31) when the subject for identification being a cow.

12. An animal species identification method as set forth in claim 9 or 10, further comprising:
identification of a horse by determining the presence or absence of at least either one peptide fragment among the peptide fragments of amino acids with sequences of GEPGPTGLPGPPGER (SEQ ID NO: 2), SGDRGEAGPAGPAGPIGPVGAR (SEQ ID NO: 11), GDGGPPGVTGFPGAAGR (SEQ ID NO: 24), GLPGVAGSLGEPGPLGIAGPPGAR (SEQ ID NO: 26), GEPGPVGSVGPVGAVGPR (SEQ ID NO: 28), SGQPGTVGPAGVR (SEQ ID NO: 32) and GSPGPQGPPGVPGPSGLIGITGAR (SEQ ID NO: 37) when the subject for identification being a horse.

13. An animal species identification method as set forth in claim 9 or 10, further comprising:
identification of a pig by determining the presence or absence of at least either one peptide fragment among the peptide fragments of amino acids with sequences of GETGPAGRPGEAGPPGPPGPAGEK (SEQ ID NO: 8), SGDRGETGPAGPAGPVGPVGAR (SEQ ID NO: 12), GIPGEFGLPGPAGPR (SEQ ID NO: 20), GEPGPAGSVGPAGAVGPR (SEQ ID NO: 29), GEMGPAGIPGAPGLMGAR (SEQ ID NO: 35) and GSPGPQGPPGAPGPGGISGITGAR (SEQ ID NO: 38) when the subject for identification being a pig.

14. An animal species identification method as set forth in claim 9 or 10, further comprising:
identification of a sheep by determining the presence or absence of the peptide fragment of amino acid with sequence of AGEVGPPGPPGPAGEK (SEQ ID NO: 9) when the subject for identification being a sheep.

15. An animal species identification method as set forth in claim 9 or 10, further comprising:
identification of animal species by narrowing down the subject to be identified to a cow or a deer by determining the presence or absence of the peptide fragment of amino acid with sequence of GEPGPAGAVGPAGAVGPR (SEQ ID NO: 27) when the animal species of the subject to be identified being narrowed down.

16. An animal species identification method as set forth in claim 9 or 10, further comprising:
identification of animal species by narrowing down the subject to be identified to a sheep or a goat by determining the presence or absence of at least either one peptide fragment among peptide fragments of amino acids with sequences of GEPGPVGAVGPAGAVGPR (SEQ ID NO: 30) and GFPGSDGVAGPK (SEQ ID NO: 4) when the animal species of the subject to be identified being narrowed down.

17. An animal species identification method as set forth in claim 9 or 10, further comprising:
identification of animal species by narrowing down the subject to be identified to a horse or a pig by determining the presence or absence of at least either one peptide fragment among peptide fragments of amino acids with sequences of GIPGPAGAAGATGAR (SEQ ID NO: 14) and GEQGPAGPPGFQGLPGPAGTAGEVGKPGER (SEQ ID NO: 18) when the animal species of the subject to be identified being narrowed down.

18. An animal species identification method being an identification of an animal skin derived from at least one species of animal selected from six species of animals of a cow, a pig, a sheep, a goat, a horse and a deer, against the decomposition product of animal skin-derived collagen extracted from a sample to be decomposed using trypsin, comprising:
1) a step of identifying a cow by determining the presence or absence of peptide fragments of amino acids with sequences of SEQ ID NO: 5, SEQ ID NO: 27 and SEQ ID NO: 31,
2) a step of identifying a deer by determining the presence or absence of peptide fragments of amino acids with sequences of SEQ ID NO: 27 and SEQ ID NO: 33,
3) a step of identifying a horse by determining the presence or absence of peptide fragment of amino acid with sequence of SEQ ID NO: 11 or SEQ ID NO: 28,
4) a step of identifying a pig by determining the presence or absence of peptide fragments of amino acid with sequence of SEQ ID NO: 12 or SEQ ID NO: 29,
5) a step of identifying a sheep by determining the presence or absence of peptide fragment of amino acid with sequence of SEQ ID NO: 9 and identifying a goat by determining the presence or absence of peptide fragment of amino acid with sequence of SEQ ID NO: 7.

19. An animal species identification method as set forth in either one of claims 9 to 18, further comprising:
said determining the presence or absence of peptide fragments being an identification of a peptide fragment by an analysis of an amino acid sequence by measuring the mass-to-charge ratio (m/z) of the pre-cursor ion and the product ion of the peptide fragment by the tandem mass (MS/MS) analysis employing a liquid chromatography mass spectrometer (LC-MS).

20. An animal species identification device being an identification of animal skin derived from at least one kind of animal selected from six kinds of animals of a cow, a pig, a sheep, a goat, a horse and a deer, comprising:
means of identifying the animal species by determining the presence or absence of at least any peptide fragments among peptide fragments of amino acids with sequences of SEQ ID NO: 1∼38 against the decomposition product of animal skin-derived collagen extracted from a sample to be decomposed using trypsin.

21. An animal species identification device being an identification of animal skin derived from at least one kind of animal selected from six kinds of animals of a cow, a pig, a sheep, a goat, a horse and a deer, comprising:
means of identifying the animal species by determining the presence or absence of at least either one peptide fragment among the peptide fragments of amino acids with sequences of SEQ ID NO: 5∼14, SEQ ID NO: 19∼22 and SEQ ID NO: 25∼33 against the decomposition product of animal skin-derived collagen extracted from a sample to be decomposed using trypsin.
